# EUROPEAN PATENT SPECIFICATION

(11) **EP 2 790 528 B1**
(45) Date of publication and mention of the grant of the patent: **23.05.2018**
(21) Application number: 12756894.7
(22) Date of filing: 30.08.2012
(51) Int. Cl.: A23K 20/174, A23K 20/105, A23K 50/40, A61K 8/365, A61K 8/02, A61K 6/00, A61Q 11/00

(54) **LACTIC ACID FOR USE IN PREVENTING DENTAL CONDITIONS**
MILCHSÄURE ZUR VERWENDUNG BEI DER PRÄVENTION VON ZAHNLEIDEN
ACIDE LACTIQUE UTILISÉ POUR PRÉVENIR DES CONDITIONS DENTAIRES

(30) Priority: 13.12.2011 US 201161570121 P
(43) Date of publication of application: 22.10.2014
(73) Proprietor: Hill's Pet Nutrition, Inc., Topeka, KS 66603 (US)
(72) Inventor: SCHERL, Dale Scott, Lawrence, Kansas 66049 (US); JEWELL, Dennis, Lawrence, Kansas 66049 (US); TOLL, Philip W., Valley Falls, Kansas 66088 (US); HAHN, Kevin A., Topeka, Kansas 66610 (US)
(74) Representative: Wichmann, Hendrik
(86) International application number: PCT/US2012/053038
(87) International publication number: WO 2013/089842

(56) References cited:
- WO-A2-2006/069241
- DE-A1- 3 104 189
- DE-A1-102008 014 224
- US-A1- 2004 115 247
- US-A1- 2004 241 109
- US-A1- 2007 148 104
- DATABASE WPI Week 200780 Thomson Scientific, London, GB; AN 2007-864289 XP002685234, & JP 2007 126409 A (SHOWA YAKUHIN KAKO KK) 24 May 2007 (2007-05-24)

## Description

### CROSS REFERENCE TO RELATED APPLICATIONS

This application claims priority to U.S. Provisional Application No. 61/570,121, filed on 13 December 2011.

### FIELD OF THE INVENTION

The present invention relates to compositions useful for preventing dental conditions in an animal, particularly in a companion animal.

### BACKGROUND OF THE INVENTION

Virtually all domestic companion animals, *e.g*., canine and feline companion animals, as well as many other animals, accumulate dental plaque and calculus (tartar). These accumulations can lead to the animal's affliction with gingivitis, which typically progresses into periodontitis that, in turn, leads to serious systemic diseases and conditions. Animals afflicted with these dental conditions are also generally afflicted with halitosis and stomatitis.

Dental plaque is formed as a result of the bacterial colonization of teeth. Dental plaque is a layer of bacteria on the erupted surfaces of teeth and in the gingival crevice. More specifically, dental plaque is a biofilm, usually a pale yellow that develops naturally on the teeth. Like any biofilm, dental plaque is formed by colonizing bacteria trying to attach themselves to a smooth surface, *e.g*., a tooth. However, the presence of plaque comprising specific bacterial species can lead to the development of dental caries and periodontal disease.

Dental calculus, or tartar, is a recurring calcified deposit on the surfaces of the teeth of many animals, including domesticated dogs and cats, humans, and primates. It is generally recognized that dental calculus develops in a sequential process that involves the accumulation of dental plaque and the subsequent calcification of the plaque by saliva, which contains very high concentrations of calcium and phosphate.

Thus, calculus or tartar is a form of hardened dental plaque, caused by the continual accumulation of minerals from saliva or plaque on the teeth. The rough surface of tartar provides an ideal medium for further plaque formation, threatening the health of the gingiva.

Brushing and flossing can remove plaque from which calculus forms; however, once formed, it is too hard and firmly attached to be removed with a toothbrush. The accumulation of plaque and its sequential solidification or calcification as dental calculus or tartar eventually causes the gingiva to become irritated and inflamed, *i.e*., the condition referred to as gingivitis. Gingivitis in turn can progress and develop into periodontitis, which is characterized by a loss of the connective tissue fibers that attach the gums to the teeth and bone that surrounds the tooth. Calculus (tartar) is detrimental to gingival health because it serves as a substrate for increased plaque formation and retention.

Once formed, calculus deposits can only be removed through dental prophylaxis or other mechanical procedures. Thus, the treatment of dental tartar is of importance not only for cosmetic reasons, but also because its role in the development of gingivitis, stomatitis, periodontitis, and the resultant systemic infections, alveolar bone recession, interference in the normal mastication process, tooth loss and adverse mouth and breath odors.

Since the microorganisms comprising dental plaque are recognized as the etiological agents responsible for the development of tartar and the subsequent inflammation of the oral soft tissues (*e.g*., gingivitis), it is well accepted that measures that remove tartar or inhibit or prevent formation of dental tartar will reduce gingivitis, and thereby obviate or inhibit the progression from tartar accumulation to gingivitis, and ultimately to periodontitis.

It is also apparent that compositions or methods that might be available for treatment of dental conditions in humans are not directly applicable for use in animals in light of, *inter alia*, the differences in compliance, cooperation, and temperament between animals, including companion canine and feline animals, and humans. Accordingly, a need exists for methods that can effectively control, reduce, or inhibit formation of dental plaque and calculus in animals. Additionally, a need exists for food products, compositions, solutions or methods that are useful for the effective control, reduction, or inhibition of formation of dental plaque and calculus in animals.

US2007148104 discloses edible chew articles for animals that comprise: (a) edible chew base, (b) an effective amount of antimicrobial lipid, and (c) at least one of (1) an effective amount of tartar control agent or (2) an effective amount of a long chain fatty acid.

JP2007126409 discloses a dental calculus dissolution agent which contains lactic acid as an active component.

WO2006069241 discloses methods for promoting oral health in an animal by causing the animal to ingest a composition comprising an oral health promoting amount of at least one antioxidant such as the antioxidants vitamin C, vitamin E, or a combination thereof.

DE102008014224 discloses a remineralizing chew toy, useful for jaw-training, tooth-cleaning and/or gum massage in animals and for preventing periodontitis.

US2004241109 discloses an oral care composition including a synergistically effective combination of (i) at least one essential oil selected from thymol, menthol, eucalyptol, methyl salicylate, anethol and eugenol, and (ii) at least one lactic acid ingredient selected from lactic acid and salts of lactic acid.

DE3104189 discloses the use of dextrorotary lactic acid for controlling diseases of the teeth.

US2007251465 discloses a pet container that has a base having at least two legs.

### BRIEF SUMMARY OF THE INVENTION

The present invention provides
lactic acid for use in preventing a dental condition in an animal, wherein the animal is a canine or a feline, wherein the dental condition is selected from dental plaque, calculus, tooth staining, halitosis, stomatitis, gingivitis, periodontitis and combinations thereof,
wherein the lactic acid is provided in a dry food composition in an amount from 1 weight % to 1.5 weight %,
wherein the food composition is a nutritionally complete companion animal pet food,
wherein the food composition further comprises from 10% to 50% protein, from 4% to 25% fat, from 20% to 70% carbohydrate, and from 4% to 20% crude fiber, and
wherein the use comprises feeding the animal the food composition.

In certain embodiments of the invention,
the composition comprises 1.0 to 1.2% lactic acid. In still a further aspect, the composition comprises 1.1 to 1.5% lactic acid.

In other embodiments of the invention,
the composition comprises
from 1.0% to 1.5%,
lactic acid, 1.0% to 1.2% lactic acid, or 1.1% to 1.5% lactic acid, and further comprises an antioxidant.

The animal in need of prevention of dental conditions is a companion animal. In one aspect, the animal is a canine while in another, the animal is a feline

The composition comprising from 1.0% to 1.5% lactic acid, 1.0% to 1.2%, or 1.1% to 1.5% lactic acid, is a companion animal pet food composition in particular it is a nutritionally-complete companion animal pet food composition.

Also disclosed herein are pet food compositions that are suitable for preventing a dental condition in an animal, *e.g.*, pet food compositions that are suitable for use in the methods disclosed herein. The dental condition is selected from the group consisting of dental plaque, calculus, tooth staining, halitosis, stomatitis, gingivitis, periodontitis and combinations thereof. The animal in need of prevention in a dental condition is a companion animal. In one aspect, the animal is a canine while in another, the animal is a feline.

The pet food compositions of the present disclosure are nutritionally complete companion animal pet food compositions comprising from 1.0% to 1.5% lactic acid, from 10% to 50% protein, from 4% to 25% fat, from 20% to 70% carbohydrate, and from 4% to 20% crude fiber.

In another specific embodiment, a pet food composition of the present disclosure also comprises an antioxidant, which, for example, can be antioxidant is selected from the group consisting of vitamin C, vitamin E, vitamin A, lipoic acid, astaxanthin, beta-carotene, L-carnitine, coenzyme Q10, glutathione, lycopene, lutein, N-acetylcysteine, soy isoflavones, S-adenosylmethionine, taurine, tocotrienols, spinach, tomato, citrus fruit, grape, carrot, broccoli, green tea, ginkgo biloba, corn gluten meal, rice bran, algae, curcumin, marine oil, fruits, vegetables, yeast, carotenoids, flavonoids, polyphenols, and mixtures thereof.

The compositions can be fed to the animal over a period of at least one month, at least two months, at least three months, at least six months or at least twelve months.

### DETAILED DESCRIPTION OF THE INVENTION

The following description of the preferred embodiment(s) is merely exemplary in nature and is in no way intended to limit the invention, its application, or uses.

As used herein and in the appended claims, the singular forms "a," "an," and "the" include plural references unless the context clearly dictates otherwise.

The term "animal" as used herein refers to non-human animals, such as mammals and is particularly applicable to companion animals. Non-human mammals include non-human primates such as monkeys, chimpanzees, *etc.* Farm animals include goats, sheep, swine, cattle, *etc.* Wild and zoo animals include wolves, bears, deer, giraffes, elephants, *etc.* Non-mammalian animals include birds and working animals include horses.

The term "companion animal" used in the present invention includes any non-human animal suitable for being kept as a pet by humans including a dog and a cat. The compositions of the invention are for the treatment of cats and/or dogs.

The term "dog" includes those dogs which are companion animals such as Canis familialis, working dogs and the like. The term dog is synonymous with the term canine.

The term "cat" includes those cat, which are companion animals known as domestic cats or house cats. The term cat is synonymous with the term feline..

As used herein, "an amount effective," "an effective amount," and like terms refer to that amount of a compound, material or composition as described herein that may be effective to achieve a particular biological result, *e.g*., to treat or reduce a dental condition selected from the group consisting of dental plaque, calculus, tooth staining, gingivitis, periodontitis, stomatitis, halitosis, and combinations thereof. Such results may be achieved, for example, by administration of the compositions of the present invention to an animal. An effective amount may be based on several factors, including the particular animal's sex, age, and weight.

The methods disclosed herein are effective to reduce accumulation of dental plaque or calculus. Accordingly, in other embodiments the method is effective to promote gingival and periodontal health as well.

The present disclosure may be suitable to any animal, *e.g*., to a mammal and more specifically to a companion animal that may benefit from feeding the formulations disclosed herein. The term "companion animal" refers to any animal that lives in close association with humans and includes, but is not limited to, canines and felines. For example, it is contemplated herein that this term may also encompass any animal whose diet may be controlled by humans and which may benefit from feeding the formulations disclosed herein. These animals may include, *e.g.,* domesticated farm animals (*e.g.* cattle, horses, swine, *etc*.) as well as undomesticated animals held in captivity, *e.g.* in zoological parks and the like. In a specific embodiment, the animal is a feline or canine.

The present invention may be suitable for use with animals in various stages in life, including lactation, weaning, growth, adult; senior, and geriatric, wherein the animal is a canine or a feline as defined in claim 1. In specific embodiments, the canine or a feline is an adult, senior, or geriatric animal, and particularly, an adult animal.

The term "treating" in its various grammatical forms in relation to the present disclosure, refers to preventing, curing, reversing, attenuating, alleviating, ameliorating, minimizing, suppressing or halting the deleterious effects of one or more of the dental conditions disclosed herein.

Unless otherwise specified, all percentages and amounts expressed herein and elsewhere in the specification should be understood to refer to percentages by weight. The amounts given are based on the active weight of the material. Except to the extent stated otherwise, all percentages used in this specification are weight percentages on a dry matter basis. The phrase "dry matter basis" means the component concentration in the composition after any moisture in the composition is removed.

Issues related to oral health are some of the most common diagnoses made by small animal veterinarians in the United States; in fact, dental calculus and gingivitis are the two most common diagnoses in canines and feline. A common etiological feature of calculus and gingivitis is bacterial plaque which if left to accumulate and solidify can lead to periodontal disease progression resulting in tissue destruction, loss of functionality, tooth loss, and the potential for systemic infection that can ultimately affect overall health. The methods and compositions disclosed herein interfere with early steps in the underlying, progressive cascade of events and factors leading to dental conditions that include dental plaque, calculus, tooth staining, halitosis, stomatitis, gingivitis, periodontitis.

Applicants have discovered that feeding canines compositions of the present disclosure comprising a high level of lactic acid inhibited both formation of dental plaque, calculus and dental stains. In particular, it has been discovered that feeding canines a pet food composition comprising 1.1% to 1.5% lactic acid and feeding felines a composition comprising
1.0% to 1.2% lactic acid inhibited formation of dental plaque, calculus and dental stains. Accordingly, in view of the above and the progressive nature of the identified dental conditions, the disclosed use of lactic acid for preventing tartar formation and tooth staining are also applicable to prevent halitosis, stomatitis, gingivitis, periodontitis, and combinations thereof.

The methods of the disclosure can be directed to treating or reducing a dental condition in an animal in need of such treatment or reduction, comprising feeding that animal a composition comprising at least 0.8% lactic acid. In other aspects the composition comprises from 0.8% to 5% lactic acid; In further aspects
the composition comprises from 1.0% to 1.9%, from 1.1% to 1.8% lactic acid, from 1.2% to 1.7% lactic acid; or about 0.8%, about 0.9%, about 1.0%, about 1.1%, about 1.2%, about 1.3%, about 1.4%, about 1.5%, about 1.6%, about 1.7%, 1.8%, 1.9%, or about 2.0% lactic acid. The composition comprises 1.0% to 1.2% lactic acid.
The composition comprises 1.1% to 1.5% lactic acid.

The dental condition to be prevented is selected from the group consisting of dental plaque, calculus, tooth staining, halitosis, stomatitis, gingivitis, periodontitis and combinations thereof. In a particular aspect, the dental condition to be prevented
is dental plaque. In another aspect. the dental condition to be prevented is calculus. In another aspect, the dental condition to be prevented is tooth staining. In a further aspect, the dental condition to be prevented is halitosis. In a still further aspect, the dental condition to be prevented is stomatitis. In one certain aspect, the dental condition to be
prevented is gingivitis, while in another certain aspect, the dental condition to be
prevented is periodontitis.

Contemplated compositions suitable for ingestion by a companion animal include, for example, foods, supplements, treats, snacks and toys

The composition comprising lactic acid is fed to the animal as a component of its food intake. The food intake of the animal can meet its ordinary nutritional requirements, which a skilled artisan can determine based upon the animal's species, age, sex, weight, and other factors. For example, a typical diet for a canine of 1-6 years of age contains on a dry matter basis about 23% protein; about 15% fat, about 0.6% phosphorus, 0.6% calcium and about 0.3% sodium; and, for older ("mature") canines and felines, a typical diet can be, for example, as provided in Table 1.

**TABLE 1**

| Typical Composition Of Diet For Mature Canines And Felines | | |
|---|---|---|
| Component (% Dry Matter) | Canine | Feline |
| Crude protein | 15 - 25 | 25 - 50 |
| Crude Fat | 7-20 | 10-30 |
| Crude Fiber | > 2 | <10 |
| Carbohydrate | 20 - 70 | 10 *-* 50 |
| Calcium | 0.5 - 1.2 | 0.6 - 1.5 |
| Phosphorous | 0.25 - 1.2 | 0.5 - 1.5 |
| Sodium | 0.15 - 0.5 | 0.15 - 0.5 |
| Magnesium | 0.05 - 0.2 | 0.05 - 0.15 |
| Energy density¹ | 3.0 - 4.5 | 3.0 - 5.0 |

| | | |
|---|---|---|
| ¹kCal ME (metabolizable energy) per kg food (dry matter) | | |

In specific embodiments, the use of lactic acid is preventing a dental condition in an animal in need comprising feeding that animal a composition comprising 1.0% to 1.2% or 1.0.% to 1.5% lactic acid, wherein the composition is a nutritionally complete companion animal pet food composition. In specific embodiment, the composition comprises 1.0% to 1.2% lactic acid. In another specific embodiment, the composition comprises 1.1% to 1.5% lactic acid. The companion animal pet food composition comprises suitable sources of protein, carbohydrate, fat, and fiber.

In a particular aspect the composition of the disclosure, comprises from 10% to 50%, from 15% to 40%, and from 20% to 30% protein. In a specific aspect, the composition of the disclosure, which composition is a companion animal pet food composition comprises about 25% protein.

In a particular aspect the composition of the disclosure, comprises from 30% to 70%, from 35% to 65%, and from 40% to 60% carbohydrate. In a specific aspect, the composition of the disclosure, which composition is a companion animal pet food composition comprises about 50% carbohydrate.

In still another aspect the composition of the disclosure, which composition is a companion animal pet food composition comprises from 4% to 20%, from 5% to 16%, from 6% to 14%, and from 8% to 12% fat. In a specific aspect, the composition of the disclosure, which composition is a companion animal pet food composition comprising about 10% fat.

In a another aspect the composition of the disclosure, comprises from 4% to 20%, from 6% to 18%, from 8% to 16% protein, and from 10% to 14% crude fiber. In a specific aspect, the composition of the disclosure, which composition is a companion animal pet food composition comprising about 12% crude fiber.

In a particular aspect the composition of the disclosure, comprises about 33% protein, about 30% carbohydrate, about 20% fat, about 8% crude fiber and 1.0% to 1.2% lactic acid.

In another particular aspect the composition of the disclosure; comprises about 25% protein, about 50% carbohydrate, about 10% fat, about 12% crude fiber and 1.1% to 1.5% lactic acid.

The compositions can be fed to the animal over a period of at least one month, at least two months, at least three months, at least six months or at least twelve months.

The pet food compositions disclosed above are particularly suitable for feeding to canines in need of dental treatment. In other embodiments, pet food compositions particularly suitable for use in the disclosed methods of treatment of feline companion animals generally have some higher levels of protein but lower levels of carbohydrate and fiber than described above.

The methods of the disclosure can be directed to treating
or reducing a dental condition in an animal in need of such treatment or reduction, comprising feeding that animal, which can be a feline companion animal, a composition comprising at least 0.8% lactic acid, from about 0.8% to about 5% lactic acid, about 1.0% to about 1.2% or about 1.1% to about 1.5% lactic acid, wherein the composition is a companion animal pet food composition. In certain aspects the compositions fed to the animal in need thereof comprise from 1.0% to 1.9%, from 1.1% to 1.8% lactic acid, from 1.2% to 1.7% lactic acid; or about 1.0%, about 1.1%, about 1.2%, about 1.3%, about 1.4%, about 1.5%, about 1.6%, about 1.7%, 1.8%, 1.9%, or about 2.0% lactic acid. In one specific embodiment, the composition comprises 1.0% to 1.2% lactic acid. In another specific embodiment, the composition comprises about 1:1% to about 1.5% lactic acid. In one aspect of such embodiments, the companion animal pet food composition comprises suitable sources of protein, carbohydrate, fat, and fiber.

In a particular aspect the methods of the disclosure comprise feeding a composition of the disclosure, which composition is a companion animal pet food composition comprising from 20% to 45%, from 25% to 40%, and from 30% to 35% protein. In a specific aspect, the methods of the disclosure comprise feeding a composition of the disclosure, which composition is a companion animal pet food composition comprising about 33% protein.

In a particular aspect the methods of the disclosure comprise feeding a composition of the disclosure, which composition is a companion animal pet food composition comprising from 15% to 45%, from 20% to 40%, and from 25% to 35% carbohydrate. In a specific aspect, the methods of the disclosure comprise feeding a composition of the disclosure, which composition is a companion animal pet food composition comprising about 30% carbohydrate.

In still another aspect the methods of the disclosure comprise feeding a composition of the disclosure, which composition is a companion animal pet food composition comprising from 5% to 35%, from 10% to 30%, and from 15% to
25% fat. In a specific aspect, the methods of the disclosure comprises feeding a composition of the disclosure, which composition is a companion animal pet food composition comprising about 20% fat.

In a another aspect the methods of the disclosure comprise feeding a composition of the disclosure, which composition is a companion animal pet food composition comprising from 4% to 15%, from 5% to 12%, from 6% to 10% protein, and from 7% to 9% crude fiber. In a specific aspect, the methods of the disclosure comprise feeding a composition of the disclosure, which composition is a companion animal pet food composition comprising about 8% crude fiber.

In a particular aspect of
the composition of the disclosure, which composition is a companion animal pet food composition comprising about 33% protein, about 30% carbohydrate, about 20% fat, about 8% crude fiber and about 1.0% to about 1.2% lactic acid.

In one illustrative example, a nutritionally-complete companion animal pet food composition for canine companion animals may further comprise, *inter alia,* vitamins, minerals and other additives. In one aspect this pet food composition may comprise at least 0.8% lactic acid, 0.8% to 5% lactic acid, from 1.0% to 1.9%, from
1.1% to 1.8% lactic acid, from 1.2% to 1.7% lactic acid; or about 0.9%, about 1.0%, about 1.1%, about 1.2%, about 1.3%, about 1.4%, about 1.5%, about 1.6%, about 1.7%, 1.8%, 1.9%, or about 2.0% lactic acid, about 1.0% to about 1.2% or about 1.1% to about 1.5% lactic acid, and may further comprise whole grain corn, soybean mill run, chicken by-product meal, powdered cellulose, corn gluten meal, soybean meal, chicken liver flavor, soybean oil, flaxseed, caramel color, iodized salt, L-lysine, choline chloride, potassium chloride, vitamin E supplement, vitamins (L-ascorbyl-2-polyphosphate (source of vitamin C), vitamin E supplement, niacin, thiamine mononitrate, vitamin A supplement, calcium pantothenate, biotin, vitamin B₁₂ supplement, pyridoxine hydrochloride, riboflavin, folic acid, vitamin D₃ supplement), minerals (ferrous sulfate, zinc oxide, copper sulfate, manganous oxide, calcium iodate, sodium selenite), taurine, L-carnitine, preserved with mixed tocopherols and citric acid, phosphoric acid, beta-carotene, and rosemary extract.

In another illustrative example, a nutritionally complete companion animal pet food composition for feline companion animals may further comprise, *inter alia,* vitamins, minerals and other additives. In one aspect this pet food composition may comprise at least 0.8% lactic acid, 0.8% to 5% lactic acid, 1.0% to 1.2% or 1.1 % to 1.5% lactic acid and may further comprise brewers rice, corn gluten meal, chicken by-product meal, powdered cellulose, whole grain corn, chicken liver flavor, animal fat (preserved with mixed tocopherols and citric acid), soybean mill run, calcium sulfate, choline chloride, potassium chloride, iodized salt, taurine, DL-methionine, vitamin E supplement, vitamins (L-ascorbyl-2-polyphosphate (source of vitamin C); vitamin E supplement, niacin, thiamine mononitrate, vitamin A supplement, calcium pantothenate, riboflavin, biotin, vitamin B₁₂ supplement, pyridoxine hydrochloride, folic acid, vitamin D₃ Supplement), minerals (ferrous sulfate, zinc oxide, copper sulfate, manganous oxide, calcium iodate, sodium selenite), L-carnitine, preserved with mixed tocopherols and citric acid, phosphoric acid, beta-carotene, and rosemary extract.

In another embodiment, the composition of the disclosure, which composition is a companion animal pet food composition as described above, further comprises an anti-oxidant. In particular, such compositions comprise an oral health-promoting effective total amount of at least one antioxidant. In certain aspects, the antioxidant is selected from the group consisting of vitamin C, vitamin E, vitamin A, lipoic acid, astaxanthin, beta-carotene, L-camitine, coenzyme Q10, glutathione, lycopene, lutein, N-acetylcysteine, soy isoflavones, S-adenosylmethionine, taurine, tocotrienols, spinach, tomato, citrus fruit, grape, carrot, broccoli, green tea, ginkgo biloba, corn gluten meal, rice bran, algae, curcumin, marine oil, fruits, vegetables, yeast, carotenoids, flavonoids, polyphenols, and mixtures thereof.

The antioxidant-comprising composition can comprise vitamin E, vitamin C, or both vitamin E and vitamin C. In one aspect, the vitamin E content of a composition can be at least about 100 ppm, illustratively about 100 to about 5000 ppm, about 250 to about 2500 ppm, or about 500 to about 1500 ppm. In another aspect
, the vitamin C content of a composition can be at least about 10 ppm, illustratively about 10 ppm to about 10,000 ppm, or about 20 to about 2000 ppm, or about 25 to about 500 ppm.

The compositions of the present invention are formulated as companion animal pet food compositions, the pet food compositions are nutritionally complete pet food compositions.

In particular embodiments, the pet food compositions, and particularly the nutritionally-complete pet food compositions of the present disclosure, can be prepared in a dry form using conventional processes. In one contemplated embodiment, dry ingredients, including, for example, animal protein sources, plant protein sources, grains, *etc.,* are ground and mixed together. Moist or liquid ingredients, including fats, oils, animal protein sources, water, *etc.,* are then added to and mixed with the dry mix. The mixture is then processed into kibbles or similar dry pieces. Kibble is often formed using an extrusion process in which the mixture of dry and wet ingredients is subjected to mechanical work at a high pressure and temperature, and forced through small openings and cut off into kibble by a rotating knife. The wet kibble is then dried and optionally coated with one or more topical coatings which can include, for example, flavors, fats, oils, powders, and the like. Kibble also can be made from the dough using a baking process, rather than extrusion, wherein the dough is placed into a mold before dry-heat processing. Kibble also can be made from a food matrix undergoing pelletization. It particular aspects of this embodiment, lactic acid is incorporated into the food composition by adding it to the above-described mixtures before extrusion or by coating the extruded kibble or pellets with, for example, lactic acid as an ingredient of a topical coating.

In another aspect of this embodiment, lactic acid is also incorporated into the food composition by adding it to the above-described mixtures before extrusion or by coating the extruded kibble or pellets with, for example, the lactic acid as an ingredient of a topical coating.

Treats can be prepared by, for example, an extrusion or baking process similar to those described above for dry food. Other processes also can be used to either apply a coating comprising lactic acid on the exterior of existing treat forms, or injecting lactic acid into an existing treat form. In a similar manner, treats of the present disclosure may also comprise one or more oxidants that may also be included in a coating comprising the exterior of existing treat forms, or injected into an existing treat form.

The composition may be a food supplement comprising lactic acid. Supplements include, for example, a feed or pet food used with another feed or pet food to improve the nutritive balance or performance of the total. Contemplated supplements include compositions that are fed undiluted as a supplement to other feeds or pet foods, offered free choice with other parts of an animal's ration that are separately available, or diluted and mixed with an animal's regular feed or pet food to produce a complete feed or pet food. Supplements can be in various forms including, for example, powders, liquids, syrups, pills, encapsulated compositions, *etc.*

The composition may be a treat comprising lactic acid. Treats include, for example, compositions that are given to an animal to entice the animal to eat during a non-meal time. Treats can be nutritional, wherein the composition comprises one or more nutrients, and can, for example, have a composition as described above for food. Non-nutritional treats encompass any other treats that are non-toxic. A lactic acid containing composition can, for example, be coated onto the treat, incorporated into the treat, or both.

The composition may be a toy comprising lactic acid. Toys include, for example, chewable toys. Contemplated toys for dogs include, for example, artificial bones. The lactic acid or lactic acid containing composition, for example, can be present in a coating on the surface of the toy or on the surface of a component of the toy, or can be incorporated partially or fully throughout the toy, or both. Lactic acid is orally accessible by the intended user.

It should be recognized that this disclosure may contemplate both partially consumable toys (*e.g*., toys comprising plastic components) and fully consumable toys (*e.g*., rawhides and various artificial bones). It should be further recognized that this disclosure may contemplate toys for
companion, farm, and zoo animal use, and particularly for canine or feline use.

The terms "treat" and "toy" can be considered interchangeable for the purposes of this specification. However, in general a treat is fully edible and a toy
has an edible coating.

In preparing a composition lactic acid can, for example, be incorporated into the composition during formulation processing, such as during and after mixing of other components of the composition. Distribution of these components into the composition can be accomplished by any conventional method including standard mixing procedures.

The compositions of the present disclosure, whether a companion animal pet food composition, dietary supplement, treat or toy, may further comprise at least one of a commercial liquid palatant enhancer or other flavor composition to create a novel flavor palatant which can then be included within or topically applied to the composition. Suitable commercial liquid palatant enhancers for use with compositions of the present disclosure can include any known or commercially available liquid palatant enhancers commercially available from pet food palatant enhancer or other flavor suppliers known to those of skill in the art. In one illustrative aspect, the kibble, treat, or toy can be coated with a composition comprising lactic acid and a carrier, wherein the carrier comprises protease-treated minced animal by-product, amino acids, one or more reducing sugars and thiamin.

As used throughout, ranges are used as shorthand for describing each and every value that is within the range. Any value within the range can be selected as the terminus of the range. In the event of a conflict in a definition in the present disclosure and that of a cited reference, the present disclosure controls.

### EXAMPLES

### Example 1: Lactic Acid Inhibition of Dental Plaque, Calculus, and Staining in Felines (Cats): Twelve Month Feeding Study

This study was designed to assess the effect of lactic acid on oral substrate accumulation (dental plaque, calculus, and tooth stain) in cats. Initially, all cats received a professional dental prophylaxis at this baseline using standard, well-established procedures. Six and twelve months later, dental substrate accumulation (dental plaque, calculus, and tooth stain) was quantified using the Logan-Boyce quantification method (*See* Logan EI, Boyce. Oral health assessment in dogs: parameters and methods. J. Vet Dent; 11(2):58-63.)

Plaque is measured by applying a disclosing solution (2% aqueous eosin) to the animal's teeth. The teeth to be scored are divided into gingival and occlusal halves, and each half is assigned a separate numerical score for plaque coverage (percentage of the tooth area stained) and thickness (intensity of staining). The score for each tooth half is calculated by multiplying the coverage and thickness scores. The gingival and occlusal scores are then added together for a tooth score. The sum of the teeth scores is identified as the total tooth score, and the whole mouth mean score is calculated by averaging the total teeth scores for each animal. The plaque scoring method gives a number of 0-4 by the percentage of plaque coverage with 0 = no plaque detected, 1 = 1-24% coverage, 2 = 25-49% coverage, 3 = 50-74% coverage and 4 = 75-100% coverage. Plaque thickness is given a score of 1-3 with 1 = pink to light red thickness, 2 = medium to red thickness and 3 = dark red. The total range for the score is 0-24, e.g., each gingival and occlusal halves = plaque coverage * plaque thickness, and then each half is added together. The whole mouth score is calculated by adding the teeth scores and dividing by the number of teeth scored.

Calculus is measured by air-drying the teeth to be scored, dividing those teeth vertically into mesial, buccal, and distal thirds, and assigning each third a numerical score based on the percentage of calculus coverage with 0 = no calculus detected, 1 = 1-24% coverage, 2 = 25-49% coverage, 3 = 50-74% coverage, and 4 = 75-100% coverage. Scores for each of the three tooth surfaces are added together for a tooth score and the total tooth score is divided by the number of teeth scored. The total range for the score is 0-12, e.g., each mesial, buccal and distal third = calculus coverage, and then each third is added together. The whole mouth score is calculated by adding the teeth scores and dividing by the number of teeth scored.

Dental stain is measured by dividing the teeth to be scored vertically into thirds as with the calculus index and given a numerical score for percentage of stain coverage and one for perceived stain intensity. The percentage of stain coverage is given a score of 0-4 with 0 = no stain detected, 1 = 1-24% coverage, 2 = 25-49% coverage, 3 = 50-74% coverage and 4 = 75-100% coverage. The intensity is given a score of 1-3 with 1 = to light yellow, tan coloring, 2 = moderate brown coloring and 3 = dark brown, black coloring. The score for each tooth (mesial, buccal and distal) is calculated by multiplying the coverage and intensity scores, and the resulting numbers are added to obtain a tooth score. The total range for the score is 0-36, e.g., each mesial, buccal and distal third = stain coverage * intensity, and then each third is added together. The whole mouth score is calculated by adding the teeth scores and dividing by the number of teeth measured to obtain the whole mouth score.

A total of 24 domestic cats, ranging in age from 6 years 3 months to 14 years 1 month were enrolled in this study. As noted above, on day 1 of this feeding study, the animals were provided with dental prophylaxis and on days 168 and 337, the oral substrate accumulation was assessed.

In this study, the control feline pet food composition was a commercially available feline pet food composition, Feline Control Food 1. The same feline pet food composition was supplemented with 1.2% lactic acid to provide a pet food composition designated herein as Feline Test Food 1. The data obtained are provided in Table 2:

**TABLE 2**

| | Group Average ± SEM | | |
|---|---|---|---|
| | Plaque | Tartar | Stain |
| Feline Control Food 1 | | | |
| 6 months | 8.1 ± 1.8 | 3.7± 1.2 | 3.8 ± 1.5 |
| 12 months | 7.0 ± 0.6 | 4.5± 0.4 | 4.8± 0.5 |
| Feline Test Food 1 | | | |
| 6 months | 5.6±1.8 | 2.6 ± 1.2 | 2.4±1.1 |
| 12 months | 4.9±0.6 | 3.3±0.4 | 3.2±0.5 |
| Difference (%) | | | |
| 6 months | 31 | 31 | 37 |
| 12 months | 30 | 25 | 33 |

As shown by the reduction of scores for plaque, tartar and stain and the percent difference of the 6 month and 12 month scores, the data of Table 2 demonstrate that lactic acid inhibits formation of dental plaque, calculus and stain when a lactic acid containing composition comprising 1.2% lactic acid is fed to cats for six months.

### Example 2: Lactic Acid Inhibition of Dental Plaque, Calculus, and Staining in Canines (Dogs): 28 day Feeding Study

This study was designed to assess the effect of lactic acid on oral substrate accumulation (dental plaque, calculus, and tooth stain) in dogs. Initially, all dogs received a professional dental prophylaxis at this baseline using standard, well-established procedures. Twenty-eight days later, dental substrate accumulation (dental plaque, calculus, and tooth stain) was quantified using the Logan-Boyce quantification method as referenced and described above.

A total of 32 beagle dogs, ranging in age from 2.5 to 9 years were enrolled in this study. As noted above, on day 1 of this feeding study, the animals were provided with dental prophylaxis and on day 28, the oral substrate accumulation was assessed.

In this study, the control canine pet food composition was a commercially available canine pet food composition, Canine Control Food 1. The same canine pet food composition was supplemented with 1.5% lactic acid to provide a pet food composition designated herein as Canine Test Food 1. The analyzed amount of lactic acid for the Canine Test Food 1 was 1.1%. The Canine Control Food 1 was analyzed to have 0.2% naturally-occurring lactic acid. The data obtained are provided in Table 3:

**TABLE 3**

| | Group Average ± SEM | | |
|---|---|---|---|
| | Plaque | Tartar | Stain |
| Canine Control Food 1 | 7.0 ± 2.0 | 4.5 ± 2.1 | 5.3 ± 2.5 |
| Canine Test Food 1 | 6.7 ± 1.2 | 2.9 ± 1.3 | 3.2 ± 1.5 |
| Difference (%) | 4.9 | 35.7 | 38.7 |

As shown by the reduction of scores for plaque, tartar and stain and the percent difference of the control versus the test food, the data of Table 3 demonstrate that lactic acid inhibits tooth calculus and stain when a lactic acid containing dog food comprising 1.5% lactic acid is fed to dogs for one month.

### Example 3: Lactic Acid Inhibition of Dental Plaque, Calculus, and Staining in Felines (Cats): Three Month Feeding Study

This study was designed to assess the effect of dietary lactic acid on oral substrate accumulation (dental plaque, calculus, and tooth stain), and to document evidence of bacteria. A calibration study was run prior to the study to ensure that high and low dental plaque formers were evenly distributed between the test and control groups. Initially, all cats received a professional dental prophylaxis at this calibration baseline using standard, well-established procedures. Twenty eight days later, dental substrate accumulation (dental plaque, calculus, and tooth stain) was quantified using the Logan-Boyce quantitation method as referenced and described above. Cats were then assigned to a test or control group based on dental plaque accumulation. One control group (fed a food without lactic acid supplementation) and two test groups (fed a food with lactic acid supplementation) were used; and each group had fifteen cats.

Since it was necessary to house the cats according to assigned group, a two-week acclimation period was used to allow the cats to get used to one another. Following this acclimation period, cats were given a second professional dental prophylaxis to establish a testing period baseline. After the prophylaxis, quantitative light-induced fluorescence (QLF) methods were used to capture baseline images of specific teeth according to standard procedures. The teeth assessed using the Logan-Boyce quantification method were the maxillary third incisor, canine, first, second, third, fourth premolars and first molar, and the mandibular canine, second, third, fourth premolars, and first molar. The teeth assessed using QLF were the maxillary canine, third and fourth premolars, and the mandibular canine, third and fourth premolars, and the first molar. The testing period of this study was three months, during which dental substrate accumulation and evidence of bacteria were assessed every twenty-eight days using the Logan-Boyce and QLF methods.

The control food was a feline pet food composition without added lactic acid, which was designated Feline Control Food 2. The same feline pet food composition was supplemented with 1.2% lactic acid to provide a pet food composition designated herein as Feline Test Food 2. The analyzed amount of lactic acid for the Feline Test Food 2 for Test Group 1 was 1.1% and for Test Group 2 was 1.2%. The Feline Control Food 2 was analyzed to have 0.3% naturally-occurring lactic acid. The Control Group was fed Feline Control Food 2 and Test Groups 1 and 2 were fed Feline Test Food 2. The data obtained for dental substrate accumulation for each time period are presented below.

The data obtained at the one-month time period are provided in Table 4:

**TABLE 4**

| | Group Average ± SEM | | |
|---|---|---|---|
| | Plaque | Tartar | Stain |
| Control Group | 5.4 ± 0.4 | 2.3 ± 0.2 | 2.1 ± 0.2 |
| | | | |
| Test Group 1 | 5.4 ± 0.4 | 1.9 ± 0.2 | 1.7 ± 0.2 |
| Difference (%) | 0.0 | 19.9 | 21.8 |
| | | | |
| Test Group 2 | 4.9 ± 0.4 | 1.8 ± 0.2 | 1.7 ± 0.2 |
| Difference (%) | 8 | 21 | 20 |

The data obtained at the two-month time period are provided in Table 5:

**TABLE 5**

| | Group Average ± SEM | | |
|---|---|---|---|
| | Plaque | Tartar | Stain |
| Control Group | 8.0 ± 0.5 | 3.1 ± 0.3 | 3.0 ± 0.3 |
| | | | |
| Test Group 1 | 7.8 ± 0.5 | 2.4 ± 0.3 | 2.3 ± 0.3 |
| Difference (%) | 2 | 23 | 21 |
| | | | |
| Test Group 2 | 6.5 ± 0.5 | 2.1 ± 0.3 | 2.0 ± 0.3 |
| Difference (%) | 19 | 34 | 31 |

The data obtained at the three-month time period are provided in Table 6:

**TABLE 6**

| | Group Average ± SEM | | |
|---|---|---|---|
| | Plaque | Tartar | Stain |
| Control Group | 8.4 ± 0.6 | 3.4 ± 0.3 | 3.4 ± 0.3 |
| | | | |
| Test Group 1 | 7.6 ± 0.6 | 2.8 ± 0.3 | 2.7 ± 0.3 |
| Difference (%) | 9 | 18 | 21 |
| | | | |
| Test Group 2 | 6.9 ± 0.4 | 2.4 ± 0.3 | 2.6 ± 0.3 |
| Difference (%) | 18 | 28 | 22 |

As shown by the reduction of scores for plaque, tartar and stain and the percent difference of the control versus the test food, the data of Tables 4-6 again demonstrate that feeding cats a pet food composition comprising elevated levels of lactic acid reduced formation of dental plaque, tartar, and stain in felines (cats). Although not presented, the QLF images qualitatively indicate that teeth of the control group cats appear to have more fluorescent substrate than the teeth of cats in either of the test groups.

## Claims

1. Lactic acid for use in preventing a dental condition in an animal, wherein the animal is a canine or a feline, wherein the dental condition is selected from dental plaque, calculus, tooth staining, halitosis, stomatitis, gingivitis, periodontitis and combinations thereof,
wherein the lactic acid is provided in a dry food composition in an amount from 1 weight % to 1.5 weight %,
wherein the food composition is a nutritionally complete companion animal pet food,
wherein the food composition further comprises from 10% to 50% protein, from 4% to 25% fat, from 20% to 70% carbohydrate, and from 4% to 20% crude fiber, and
wherein the use comprises feeding the animal the food composition.

2. The lactic acid for use according to claim 1, wherein the composition comprises lactic acid in an amount from 1.0 to 1.2 weight %, or from 1.1 to 1.5 weight%.

3. The lactic acid for use according to any preceding claim, wherein the dental condition is dental plaque.

4. The lactic acid for use according to any preceding claim, wherein the dental condition is calculus.

5. The lactic acid for use according to any preceding claim, wherein the dental condition is tooth staining.

6. The lactic acid for use according to any preceding claim, wherein the dental condition is gingivitis.

7. The lactic acid for use according to any preceding claim, wherein the dental condition is periodontitis.

8. The lactic acid for use according to any preceding claim, wherein the composition further comprises at least one antioxidant, optionally wherein the antioxidant is selected from vitamin C, vitamin E, vitamin A, lipoic acid, astaxanthin, beta-carotene, L-carnitine, coenzyme Q10, glutathione, lycopene, lutein, N-acetylcysteine, soy isoflavones, S-adenosylmethionine, taurine, tocotrienols, spinach, tomato, citrus fruit, grape, carrot, broccoli, green tea, ginkgo biloba, corn gluten meal, rice bran, algae, curcumin, marine oil, fruits, vegetables, yeast, carotenoids, flavonoids, polyphenols, and mixtures thereof.

9. The lactic acid for use according to any preceding claim, wherein the animal is fed the composition for at least one month, optionally at least two months, or at least three months.

10. The lactic acid for use according to any preceding claim, wherein the composition comprises from 30% to 40% protein, from 15% to 25% fat, from 25% to 35% carbohydrate, and from 5% to 10% crude fiber, or from 20% to 30% protein, from 8% to 12% fat, from 40% to 60% carbohydrate, and from 10% to 14% crude fiber.

11. The lactic acid for use according to any of claims 1 to 10, wherein the composition comprises 1.1% lactic acid, 33% protein, 30% carbohydrate, 20% fat, and 8% crude fiber.

12. The lactic acid for use according to any of claims 1 to 10, wherein the composition comprises 1.5% lactic acid, 25% protein, 50% carbohydrate, 10% fat, and 12% crude fiber.

## Patentansprüche

1. Milchsäure zur Verwendung bei der Verhinderung eines Zahnleidens bei einem Tier, wobei das Tier ein Hund oder eine Katze ist, wobei das Zahnleiden aus Plaque, Zahnstein, Zahnverfärbung, Halitose, Stomatitis, Gingivitis, Parodontitis und Kombinationen davon ausgewählt ist,
wobei die Milchsäure in einer Trockenfutterzusammensetzung in einer Menge von 1 Gew.-% bis 1,5 Gew.-% bereitgestellt wird,
wobei die Futterzusammensetzung ein diätetisch vollständiges Haustierfutter ist,
wobei die Futterzusammensetzung weiterhin von 10 % bis 50 % Protein, von 4 % bis 25 % Fett, von 20 % bis 70 % Kohlenhydrat und von 4 % bis 20 % Rohfaser umfasst und
wobei die Verwendung das Füttern des Tiers mit der Futterzusammensetzung umfasst.

2. Milchsäure zur Verwendung nach Anspruch 1, wobei die Zusammensetzung Milchsäure in einer Menge von 1,0 bis 1,2 Gew.-% oder von 1,1 bis 1,5 Gew.-% umfasst.

3. Milchsäure zur Verwendung nach einem vorhergehenden Anspruch, wobei das Zahnleiden Plaque ist.

4. Milchsäure zur Verwendung nach einem vorhergehenden Anspruch, wobei das Zahnleiden Zahnstein ist.

5. Milchsäure zur Verwendung nach einem vorhergehenden Anspruch, wobei das Zahnleiden Zahnverfärbung ist.

6. Milchsäure zur Verwendung nach einem vorhergehenden Anspruch, wobei das Zahnleiden Gingivitis ist.

7. Milchsäure zur Verwendung nach einem vorhergehenden Anspruch, wobei das Zahnleiden Parodontitis ist.

8. Milchsäure zur Verwendung nach einem vorhergehenden Anspruch, wobei die Zusammensetzung weiterhin ein Antioxidationsmittel umfasst, gegebenenfalls wobei das Antioxidationsmittel aus Vitamin C, Vitamin E, Vitamin A, Liponsäure, Astaxanthin, Betacarotin, L-Carnitin, Coenzym Q10, Glutathion, Lycopin, Lutein, N-Acetylcystein, Sojaisoflavonen, S-Adenosylmethionin, Taurin, Tocotrienolen, Spinat, Tomate, Zitrusfrucht, Traube, Möhre, Brokkoli, grünem Tee, Ginkgo biloba, Maiskleber, Reiskleie, Algen, Kurkuma, Seetiertran, Obst, Gemüse, Hefe, Carotinoiden, Flavonoiden, Polyphenolen und Gemischen davon ausgewählt ist.

9. Milchsäure zur Verwendung nach einem vorhergehenden Anspruch, wobei das Tier für mindestens einen Monat, gegebenenfalls mindestens zwei Monate oder mindestens drei Monate mit der Zusammensetzung gefüttert wird.

10. Milchsäure zur Verwendung nach einem vorhergehenden Anspruch, wobei die Zusammensetzung von 30 % bis 40 % Protein, von 15 % bis 25 % Fett, von 25 % bis 35 % Kohlenhydrat und von 5 % bis 10 % Rohfaser oder von 20 % bis 30 % Protein, von 8 % bis 12 % Fett, von 40 % bis 60 % Kohlenhydrat und von 10 % bis 14 % Rohfaser umfasst.

11. Milchsäure nach einem der Ansprüche 1 bis 10, wobei die Zusammensetzung 1,1 % Milchsäure, 33 % Protein, 30 % Kohlenhydrat, 20 % Fett und 8 % Rohfaser umfasst.

12. Milchsäure nach einem der Ansprüche 1 bis 10, wobei die Zusammensetzung 1,5 % Milchsäure, 25 % Protein, 50 % Kohlenhydrat, 10 % Fett und 12 % Rohfaser umfasst.

## Revendications

1. Un acide lactique destiné à être utilisé pour prévenir une condition dentaire chez un animal, dans lequel l'animal est un chien ou un félin, dans lequel la condition dentaire est sélectionnée parmi la plaque dentaire, le tartre, les taches sur les dents, l'halitose, la stomatite, la gingivite, la parodontite et des combinaisons de ces derniers,
dans lequel l'acide lactique est fourni dans une composition alimentaire sèche dans une quantité comprise entre 1 % en poids et 1,5 % en poids,
dans lequel la composition alimentaire est un aliment nutritionnellement complet pour un animal de compagnie,
dans lequel la composition alimentaire comprend en outre entre 10 % et 50 % de protéines, entre 4 % et 25 % de matières grasses, entre 20 % et 70 % de glucides et entre 4 % et 20 % de fibres brutes, et
dans lequel l'utilisation consiste à nourrir l'animal avec la composition alimentaire.

2. L'acide lactique destiné à être utilisé selon la revendication 1, dans lequel la composition comprend l'acide lactique dans une quantité comprise entre 1,0 et 1,2 % en poids ou entre 1,1 et 1,5 % en poids.

3. L'acide lactique destiné à être utilisé selon l'une quelconque des revendications précédentes, dans lequel la condition dentaire est la plaque dentaire.

4. L'acide lactique destiné à être utilisé selon l'une quelconque des revendications précédentes, dans lequel la condition dentaire est le tartre.

5. L'acide lactique destiné à être utilisé selon l'une quelconque des revendications précédentes, dans lequel la condition dentaire est constituée par les taches sur les dents.

6. L'acide lactique destiné à être utilisé selon l'une quelconque des revendications précédentes, dans lequel la condition dentaire est la gingivite.

7. L'acide lactique destiné à être utilisé selon l'une quelconque des revendications précédentes, dans lequel la condition dentaire est la parodontite.

8. L'acide lactique destiné à être utilisé selon l'une quelconque des revendications précédentes, dans lequel la composition comprend en outre au moins un antioxydant, en option dans lequel l'antioxydant est sélectionné parmi la vitamine C, la vitamine E, la vitamine A, l'acide lipoïque, l'astaxanthine, le bêta-carotène, la L-carnitine, le coenzyme Q10, le glutathion, le lycopène, la lutéine, la N-acétylcystéine, les isoflavones de soja, la S-adénosylméthionine, la taurine, les tocotriénols, l'épinard, la tomate, les agrumes, le raisin, la carotte, le broccoli, le thé vert, le ginkgo biloba, la farine de gluten de maïs, le son de riz, les algues, la curcumine, l'huile marine, les fruits, les légumes, la levure, les caroténoïdes, les flavonoïdes, les polyphénols, et des mélanges de ces derniers.

9. L'acide lactique destiné à être utilisé selon l'une quelconque des revendications précédentes, dans lequel l'animal est nourri avec la composition pendant au moins un mois, en option au moins deux mois ou au moins trois mois.

10. L'acide lactique destiné à être utilisé selon l'une quelconque des revendications précédentes, dans lequel la composition comprend entre 30 % et 40 % de protéines, entre 15 % et 25 % de matières grasses, entre 25 % et 35 % de glucides et entre 5 % et 10 % de fibres brutes, ou entre 20 % et 30 % de protéines, entre 8 % et 12 % de matières grasses, entre 40 % et 60 % glucides et entre 10 % et 14 % de fibres brutes.

11. L'acide lactique destiné à être utilisé selon l'une quelconque des revendications 1 à 10, dans lequel la composition comprend 1,1 % d'acide lactique, 33 % de protéines, 30 % de glucides, 20 % de matières grasses et 8 % de fibres brutes.

12. L'acide lactique destiné à être utilisé selon l'une quelconque des revendications 1 à 10, dans lequel la composition comprend 1,5 % d'acide lactique, 25 % de protéines, 50 % de glucides, 10 % de matières grasses, et 12 % de fibres brutes.
